Europäisches Patentamt

European Patent Office

Office européen des brevets

(11) Veröffentlichungsnummer: **0 444 277 A2**

(12) **EUROPÄISCHE PATENTANMELDUNG**

(21) Anmeldenummer: 90124295.8

(22) Anmeldetag: 15.12.90

(51) Int. Cl.⁵: **C08G 61/02, C07C 17/38, C07C 39/00**

(30) Priorität: 27.02.90 DE 4006129

(43) Veröffentlichungstag der Anmeldung:
04.09.91 Patentblatt 91/36

(84) Benannte Vertragsstaaten:
**DE FR GB**

(71) Anmelder: **BAYER AG**

**W-5090 Leverkusen 1 Bayerwerk(DE)**

(72) Erfinder: **Abele, Manfred**
**Am Boerschsgarten 16**
**W-5000 Köln 90(DE)**
Erfinder: **Buysch, Hans-Josef, Dr.**
**Brandenburger Strasse 28**
**W-4150 Krefeld(DE)**
Erfinder: **Happ, Michael, Dr.**
**Göthestrasse 57c**
**W-4047 Dormagen(DE)**
Erfinder: **Obrecht, Werner, Dr.**
**Holderberger Strasse 108**
**W-4130 Moers 2(DE)**
Erfinder: **Schrage, Heinrich, Dr.**
**Doerperhofstrasse 31**
**W-4150 Krefeld 1(DE)**
Erfinder: **Vernaleken, Hugo**
**Kreuzbergstrasse 147**
**W-4150 Krefeld(DE)**

(54) **Verfahren zur Isolierung von Tetrachlorbutan und zur Herstellung von Phenolharzen aus Gemischen chlorsubstituierter Kohlenwasserstoffe.**

㊗ Bei der Chlorierung von Butadien anfallende Nebenprodukte können in Abwesenheit von Eisenkatalysatoren mit Phenolen zu Novolak-artigen Phenolharzen so umgesetzt werden, daß das in den Nebenprodukten enthaltene Tetrachlorbutan nicht reagiert. Es kann mit dem überschüssigen Phenol abdestilliert und aus dem erhaltenen Destillat in reiner Form isoliert werden`

EP 0 444 277 A2

Die Erfindung betrifft ein Verfahren, wonach aus Gemischen wenigstens teilweise chlorsubstituierter C₄- bis C₈-Kohlenwasserstoffe enthaltend u.a. 1.2.3.4-Tetrachlorbutan durch Umsetzung mit Phenolen Novolak-artige Phenolharze derart hergestellt werden, daß das 1.2.3.4-Tetrachlcrbutan nicht reagiert und isoliert werden kann. Auf diese Weise kann man Nebenprodukte, wie sie beispielsweise bei der Chlorierung von Butadien anfallen, zu zwei völlig verschiedenen wertvollen Produkten aufarbeiten und dadurch die bislang übliche Entsorgung durch Verbrennen vermeiden.

Novolake sind bekanntlich schmelzbare, in einer Reihe organischer Lösungsmittel lösliche, nicht-selbsthärtende Polyphenole, deren aromatische Kerne durch Alkylidengruppen verknüpft sind. Sie können aus Phenolen und Ketoverbindungen in Gegenwart saurer Katalysatoren hergestellt werden, wobei man üblicherweise ein Molverhältnis Ketoverbindung/Phenol von maximal 1, vorzugsweise von maximal 0,75, einhält; vgl. "Methoden der Organischen Chemie" (Houben-Weyl), Bd. 14/2, Georg Thieme Verlag, Stuttgart 1963, S. 193 f.

Unter "Novolak-artigen Phenolharzen" im Sinne der Erfindung sollen Phenolharze verstanden werden, deren verknüpfende Glieder nicht auf Alkylidengruppen beschränkt sind, die aber im wesentlichen frei von zur Selbstvernetzung befähigten Gruppen - hauptsächlich also frei von Hydroxymethylgruppen - sind.

Im Verlauf der Chloroprenherstellung durch Chlorierung von Butadien entstehen neben Chloropren unerwünschte Nebenprodukte mit 4 bis 8, vorzugsweise 4 oder 8, C-Atomen und mindestens 3, vorzugsweise mindestens 4 funktionellen Gruppen aus der Reihe bestehend aus Chloratomen und C=C-Doppelbindungen. Typische Nebenprodukte bestehen aus Mischungen aus 20 bis 60 Gew.-% 1,2,3,4-Tetrachlorbutan, 10 bis 60 Gew.-% Dichloroctadienen, 3 bis 20 Gew.-% Trichlorbutenen, 2 bis 8 Gew.-% Tetrachloroctenen und bis zu 15 Gew.-% Dichlorbutenen, Dichlorbutanen und Hexachloroctanen. Die saubere Trennung der einzelnen Komponenten aus diesen Mischungen durch Destillation ist aufgrund der nahe beieinander liegenden Siedepunkte nicht möglich; aus diesem Grund wurden die Nebenprodukte bislang nicht weiterverwendet, sondern verbrannt. Dies ist insbesondere im Hinblick auf das im Nebenproduktgemisch vorhandene 1.2.3.4-Tetrachlorbutan ein gravierender Nachteil, weil die Dehydrochlorierung dieses Produkt zu 2.3-Dichlorbutadien - einem wichtigen Comonomer für die Polychloroprensynthese - führt.

Aufgabe der Erfindung war es daher, ausgehend von dem beschriebenen Gemisch einen Weg zur Isolierung des 1.2.3.4-Tetrachlorbutans in reiner Form zu finden und für die weniger wertvollen Komponenten die wirtschaftlich und ökologisch unerwünschte Verbrennung zu vermeiden und diese Komponenten in wertvollere Produkte überzuführen.

Überraschenderweise wurde nun gefunden, daß es möglich ist, die beschriebenen Nebenprodukte mit Phenolen zu Novolak-artigen Phenolharzen so umzusetzen, daß sämtliche Komponenten mit Ausnahme des 1.2.3.4-Tetrachlorbutans reagieren. Das verbliebene Tetrachlorbutan kann, zusammen mit überschüssigem Phenol und gegebenenfalls verwendetem Lösungsmittel, destillativ abgetrennt und aus dem erhaltenen Destillat durch fraktionierte Destillation gewonnen werden; es ist dann ohne weitere Reinigung als Ausgangsmaterial für die Herstellung von 2.3-Dichlorbutadien einsetzbar. Das als Rückstand verbliebene Phenclharz kann ohne Rücksicht auf seine heterogenen Bausteine unter Verzicht auf aufwendige Reinigungsschritte weiterverwendet werden, z.B. als verstärkende Komponente in zu vulkanisierenden Kautschukmassen.

Zwar war die Umsetzung von ungesättigten Kohlenwasserstoffen mit Phenolen zu Phenolharzen aus der US-PS 3 644 537 bekannt; allerdings bestand dort nicht das Problem, aus kompliziert zusammengesetzten Mischungen chlorierter Kohlenwasserstoffe das darin vorhandene 1.2.3.4-Tetrachlorbutan zu isolieren und die anderen Komponenten der Mischungen umzusetzen.

Gegenstand der Erfindung ist also ein Verfahren zur Isolierung von 1.2.3.4-Tetrachlorbutan und zur Herstellung von Novolak-artigen Phenolharzen durch Umsetzung von A) Phenolen mit B) Gemischen wenigstens teilweise Chlor-substituierter C₄- bis C₈-Kohlenwasserstoffe, die 1.2.3.4-Tetrachlorbutan enthalten, in Abwesenheit wirksamer Mengen von Eisenkatalysatoren, wonach man nach Beendigung der Alkylierung das Phenolharz von verbliebenem 1.2.3.4-Tetrachlorbutan, überschüssigem Phenol und gegebenenfalls verwendetem Lösungsmittel auf an sich bekannte Weise trennt.

Für das erfindungsgemäße Verfahren erscheinen die oben beschriebenen Gemische chlorierter Kohlenwasserstoffe dann lohnend, wenn ihr Gehalt an 1.2.3.4-Tetrachlorbutan mindestens 10, vorzugsweise mindestens 20 Gew.-%, bezogen auf Gemisch, beträgt. Der Chlorgehalt der Gemische B beträgt in der Regel 40 bis 70, vorzugsweise 45 bis 60, Gew.-%, bezogen auf Gemisch B.

Für das erfindungsgemäße Verfahren bevorzugte Phenole A umfassen ein- und zweiwertige einkernige Phenole, die neben den phenolischen Hydroxylgruppen keine anderen Substituenten aufweisen, wie unsubstituiertes Phenol selbst, Brenzcatechin, Resorcin, Hydrochinon; einwertige C₁-C₆-Alkylphenole wie Kresole, Xylenole, Ethylphenole, Hexylphenole; einwertige Phenylphenole wie Hydroxybiphenyle; ein- und zweikerige C₆-C₁₈-Bisphenole wie Dihydroxybiphenyle, Bis-(4-hydroxyphenyl)-methan, 2,2- Bis-(4-hydrox-

EP 0 444 277 A2

yphenyl)-propan und Bis-(4-hydroxyphenyl)-sulfid.

Für das erfindungsgemäße Verfahren werden die Ausgangsprodukte in der Regel entsprechend einem Äquivalentverhältnis von phenolischem OH zu funktionellen Gruppen der Nebenproduktkomponenten (Chlor bzw. C=C-Doppelbindung) von 1:10 bis 10:1, vorzugsweise 1:4 bis 8:1, insbesondere 1:2 bis 5:1, eingesetzt.

Bevorzugte Eisenkatalysatoren, die für das erfindungsgemäße Verfahren nicht in wirksamen Mengen eingesetzt werden sollen, weil sie die Reaktion von 1.2.3.4-Tetrachlorbutan mit Phenolen katalysieren, umfassen metallisches Eisen sowie Verbindungen des Eisens, vorzugsweise des 2- und 3-wertigen Eisens, wie z.B. die Bromide, die Nitrate, die Sulfate, die Oxalate, insbesondere die Chloride. Die wirksame Menge beträgt im allgemeinen 0,05 bis 10, vorzugsweise 0,1 bis 3 Gew.-%, bezogen auf Gemische eingesetzter Kohlenwasserstoffe B.

Für das erfindungsgemäße Verfahren geeignete Katalysatoren umfassen alle chemischen Stoffe, die Friedel-Crafts-Alkylierungen beschleunigen (mit Ausnahme von Eisenkatalysatoren, s. oben), also Protonsäuren und Lewis-Säuren, wie z.B. Schwefelsäure, Chlorwasserstoff, Phosphorsäure, Aluminiumchlorid, Zinnchlorid, Bortrifluorid, Titantetrachlorid, Zinkchlorid und Zinksulfat. Zinksalze werden bevorzugt; Zinksulfat wird besonders bevorzugt. Die Reaktion kann auch ohne Katalysatorzusatz durchgeführt werden, weil der während der Reaktion entstehende Chlorwasserstoff ebenfalls katalytisch wirkt.

Übliche Katalysatormengen liegen bei Salzen im allgemeinen bei 0,05 bis 10, vorzugsweise 0,1 bis 3, Gew.-%, bei Säuren im allgemeinen bei 0,05 bis 10, vorzugsweise 0,1 bis 5 Gew.-%, jeweils bezogen auf Gemisch B.

Vorzugsweise wird das erfindungsgemäße Verfahren in Abwesenheit von Lösungsmitteln durchgeführt. Es ist jedoch ohne weiteres möglich, unter Reaktionsbedingungen inerte organische Lösungsmittel, vorzugsweise solche mit einem Siedepunkt über 120, insbesondere über 180 °C, wie Nitrobenzol, Dichlorbenzole, Benzonitril, Chlornaphthaline mitzuverwenden. Falls man das Verfahren in Lösung durch führen möchte, wird man das organische Lösungsmittel in Mengen von 5 bis 100 Gew.-%, bezogen auf die Summe von Phenol A und Gemisch B, einsetzen.

Das erfindungsgemäße Verfahren ist exotherm und kann deshalb, sobald es in Gang gekommen ist, ohne Wärmezufuhr von außen ablaufen. Um einen möglichst vollständigen Reaktionsablauf und damit einen möglichst geringen Chlorgehalt des Endprodukts zu erreichen, kann es sinnvoll sein, das Reaktionsgemisch nach Beendigung der Zugabe der Komponenten noch 2 bis 20 Stunden bei Temperaturen von 40 bis 280, vorzugsweise 80 bis 250, insbesondere 120 bis 200 °C zu belassen; das Ende der Reaktion läßt sich an der Beendigung der Chlorwasserstoffentwicklung ablesen.

Praktisch läßt sich das erfindungsgemäße Verfahren beispielsweise so durchführen, daß man das geschmolzene Phenol A und den Katalysator vorlegt und das Gemisch B, gegebenenfalls gelöst in organischem Lösungsmittel, zudosiert. Um eine gute Durchmischung der Komponenten zu erreichen, kann man rühren. Nach beendeter Reaktion kann man Lösungsmittel (falls vorhanden) und überschüssiges Phenol abtrennen, vorzugsweise durch Destillation, gegebenenfalls bei vermindertem Druck. Das Tetrachlorbutan kann dabei direkt oder aus dem erhaltenen Destillat auf an sich bekannte Weise, beispielsweise durch fraktionierte Destillation, gewonnen werden. Das abgetrennte überschüssige PHenol kann, evtl. im Gemisch mit gegebenenfalls verwendetem Lösungsmittel, für weitere Umsetzungen wiederverwendet werden.

Die erfindungsgemäß hergestellten Phenolharze enthalten pro Mol aus Phenol A stammender Einheit 0,2 bis 1, vorzugsweise 0,4 bis 0,8 Mol aus Gemisch B stammende Einheiten.

Die erfindungsgemäß hergestellten Phenolharze besitzen im allgemeinen Erweichungspunkte (nach DIN 53 244) von 50 bis 200 °C, OH-Zahlen von 100 bis 550 und als Zahlenmittel $\overline{M}_n$ bestimmte Molekulargewichte von 250 bis 2.000 (dampfdruckosmometrisch in Methanol und in Aceton bestimmt, wobei der niedrigere Wert als der korrekte angesehen wird).

Die erfindungsgemäßen hergestellten Phenolharze eignen sich als Verstärkerharzkomponente für zu vulkanisierende Kautschukmassen. Diesen Kautschukmassen können Natur- und Synthesekautschuke zugrundeliegen.

Bevorzugte Synthesekautschuke sind beispielsweise bei W.Hofmann, Kautschuk-Technologie, Gentner Verlag, Stuttgart 1980, beschrieben. Sie umfassen u.a.

| | |
|---|---|
| BR - | Polybutadien |
| ABR - | Butadien/Acrylsäure-$C_1$-$C_4$-alkylester-Copolymerisate mit Acrylester-Gehalten von 5 bis 60, vorzugsweise von 15 bis 50 Gew.-% |
| CR - | Polychloropren |
| IR - | Polyisopren |
| IIR | - Isobutylen/Isopren-Copolymerisate |

3

SBR - Styrol/Butadien-Copolymerisate mit Styrolgehalten von 1 bis 60, vorzugsweise von 20 bis 50 Gew.-%

NBR - Butadien/Acrylnitril-Copolymerisate mit Acrylnitrilgehalten von 5 bis 60, vorzugsweise 10 bis 50 Gew.-%

EPDM - Ethylen/Propylen/Dien-Copolymerisate

und Mischungen dieser Kautschuke. Die für das erfindungsgemäße Verfahren zu verwendenden Kautschuke besitzen Glasübergangstemperaturen unter 20°C, vorzugsweise unter 0°C, bestimmt im Torsionsschwingungsversuch nach DIN 53 445. Die Dosierung der Phenolharze kann 1 bis 50, vorzugsweise 3 bis 15, Gew.-%, bezogen auf Kautschuk, betragen.

Da die erfindungsgemäß hergestellten Phenolharze nicht selbsthärtend sind, bedarf es zu ihrer Härtung - wie bei den Novolaken - eines Zusatzes von Formaldehyd, Formaldehyd abspaltenden Verbindungen wie Hexamethylentetramin oder Methylolgruppen enthaltenden Melamin- oder Harnstoffkondensaten, wobei diese Härter in der Regel in Mengen von 2,5 bis 50, vorzugsweise 5 bis 15 Gew.-%, bezogen auf Phenolharz, eingesetzt werden. Werden die Phenolharze gehärtet und der sie umgebende Kautschuk vulkanisiert, so ist anzunehmen, daß vernetzte Systeme im Sinne von sog. Interpenetrating Networks entstehen. Dabei ist dann davon auszugehen, daß das erfindungsgemäß hergestellte Phenolharz nicht Teil des für den Kautschuk notwendigen Vulkanisiersystems ist.

Die Verwendung dieser Harzsysteme in Kautschukmassen führt zur Verbesserung einiger wichtiger mechanischer Eigenschaften der daraus hergestellten Vulkanisate, wie z.B. der Härte und der Spannungswerte. Die Einarbeitung der Verstärkerharze kann mittels der für die Herstellung von Kautschukmischungen üblichen Einrichtungen, z.B. mit Innenmischern und Walzwerken erfolgen. Bei hohen Mischungstemperaturen (Innenmischer) sollten zur Erzielung einer möglichst hohen Vulkanisathärte Verstärkerharz und Härter zur Vermeidung von vorzeitigen Reaktionen getrennt eingearbeitet werden. Hierbei ist es angezeigt, den Härter erst gegen Ende der Mischungsherstellung bei möglichst niedriger Mischungstemperatur (max. ca. 100°C) einzumischen.

Die erfindungsgemäßen Harze können zur Herstellung von technischen Gummiwaren, z.B. Walzen, Dichtungen, Bodenbeläge, eingesetzt werden.

Die Prozentangaben der nachfolgenden Beispiele beziehen sich jeweils auf das Gewicht; Teile sind Gewichtsteile.

Beispiele

Als "Produktgemisch" wird in den folgenden Beispielen eine Mischung von bei der Butadienchlorierung anfallenden Nebenprodukten mit einem Chlorgehalt von 54 % und einem C=C-Doppelbindungsgehalt von 0,575 Äquivalenten pro 100 g Produkt eingesetzt. Das Produktgemisch bestand zu 35 % aus Tetrachlorbutan, 40 % Dichloroctadienen, 7 % Trichlorbutenen, 8 % Tetrachloroctenen und 10 % anderen Produkten in geringeren Anteilen.

Beispiel 1

Zu einer Schmelze von 200 g Phenol und 2 g wasserfreiem Zinksulfat wurden bei 60°C 200 g Produktgemisch zugetropft, wobei sich die Reaktionsmischung auf ca. 80°C erwärmte. Anschließend wurde noch 6 Stunden auf 182°C erwärmt und das Tetrachlorbutan zusammen mit überschüssigem Phenol abdestilliert. Als Rückstand verblieben 179 g des Phenolharzes vom Erweichungspunkt 98°C; OH-Zahl: 215.

Durch fraktionierte Destillation des erhaltenen Destillats bei Normaldruck ließen sich neben 106 g eines Phenol/Tetrachlorbutan-Gemisches (94:6 Teile) 64 g reines Tetrachlorbutan gewinnen.

Beispiel 2

Zu einer Schmelze von 200 g Phenol wurden bei 60°C 200 g Produktgemisch zugetropft, wobei sich die Reaktionsmischung auf ca. 80°C erwärmte. Anschließend wurde noch 8 Stunden auf 182°C erwärmt und das Tetrachlorbutan zusammen mit dem überschüssigen Phenol abdestilliert. Als Rückstand verblieben 165 g Phenolharz vom Erweichungspunkt 70°C; OH-Zahl: 158.

Durch fraktionierte Destillation des erhaltenen Destillats bei Normaldruck ließen sich neben 127 g eines Phenol/Tetrachlorbutan-Gemisches (94:6 Teile) 62 g reines Tetrachlorbutan gewinnen.

Beispiel 3

4

Zu einer Schmelze von 200 g Phenol und 2 g Zinntetrachlorid wurden bei 60°C 200 g Produktgemisch zugetropft, wobei sich die Reaktionsmischung auf ca. 80°C erwärmte.

Anschließend wurde noch 6 Stunden auf 182°C erwärmt und das Tetrachlorbutan zusammen mit dem überschüssigen Phenol abdestilliert. Als Rückstand verblieben 179 g Phenolharz vom Erweichungspunkt 88°C; OH-Zahl: 215.

Durch fraktionierte Destillation des erhaltenen Destillats bei Normaldruck ließen sich neben 106 g eines Phenol/Tetrachlorbutan-Gemisches (94:6 Teile) 64 g reines Tetrachlorbutan gewinnen.

Beispiel 4

Zu einer Schmelze von 400 g Phenol und 4 g wasserfreiem Chromchlorid wurden bei 60°C 200 g zugetropft, wobei sich die Reaktionsmischung auf ca. 80°C erwärmte. Anschließend wurde noch 6 Stunden auf 182°C erwärmt und das Tetrachlorbutan zusammen mit dem überschüssigen Phenol abdestilliert. Als Rückstand verblieben 214 g Phenolharz vom Erweichungspunkt 69°C; OH-Zahl: 240.

Durch fraktionierte Destillation des erhaltenen Destillats bei Normaldruck ließen sich neben 283 g eines Phenol/Tetrachlorbutan-Gemisches (94:6 Teile) 53 g reines Tetrachlorbutan gewinnen.

Anwendung

Der folgende Versuch dient zur Erläuterung des Einsatzes der erfindungsgemäß hergestellten Phenolharze als Verstärkerharze für Kautschukmassen bzw. deren Vulkanisate.

Hierfür wurde folgende Prüfmischung verwendet, die zweistufig hergestellt wurde. Der erste Teil der Mischungsherstellung wurde in einem Innenmischer (Kneter) durchgeführt. Hierbei wurden folgende Bestandteile gemischt (in Teilen):

| | |
|---|---|
| Naturkautschuk (Typ SMR 5) | 75,0 |
| Polybutadien | 25,0 |
| Stearinsäure | 2,0 |
| Zinkoxid | 5,0 |
| Verstärkerharz (vgl. Tabelle) | 7,5 |
| Ruß N 326 | 70,0 |
| N-Isoproyl-N'-phenyl-p-phenylen-diamin (IPPD) | 1,5 |
| 2,2,4-Trimethyl-1,2-dihydrochinolin, polymerisiert (TMQ) | 1,0 |
| | 187,0 |

Nach einer Mischzeit von 5 Minuten wurde der Innenkneter entleert und die Teilmischung auf einem nachgeschalteten Walzwerk nach folgender Rezeptur fertiggemischt (in Teilen):

| | |
|---|---|
| Teilmischung | 187,0 |
| Schwefel | 2,5 |
| Benzothiazyl-2-sulfenmorpholid | 1,5 |
| N-Cyclohexyl-thiophthalimid | 0,3 |
| Hexamethylentetramin | 0,76 |

5

Die Vulkanisation der fertigen Mischungen erfolgte während 30 Minuten bei 150°C.

In der folgenden Tabelle werden die Ergebnisse der Vulkanisatprüfung aufgeführt. Die Ergebnisse der Vulkanisatprüfungen lassen erkennen, daß die erfindungsgemäß hergestellten Harze eine ausgezeichnete verstärkende Wirkung aufweisen.

**Vulkanisate, verstärkt mit verschiedenen Harzen**

| Prüfungen | Vergleich Verstärkerharz-freies Vulkanisat | Harz aus Beispiel 1 |
|---|---|---|
| Reißfestigkeit (MPa) | 19,4 | 14,2 |
| Bruchdehnung (%) | 332 | 290 |
| Spannungswert bei 100 % Dehnung (MPa) | 4,4 | 5,2 |
| Härte (Shore A) bei 23°C | 77 | 90 |
| bei 70°C | 72 | 88 |
| Rückprallelastizität (%) bestimmt bei 23°C | 43 | 38 |
| 70°C | 53 | 43 |

## Patentansprüche

1. Verfahren zur Isolierung von 1.2.3.4-Tetrachlorbutan und zur Herstellung von Novolak-artigen Phenolharzen durch Umsetzung von A) Phenol mit B) Gemischen wenigstens teilweise chlorsubstituierter $C_4$-bis $C_8$-Kohlenwasserstoffe, die 1.2.3.4-Tetrachlorbutan enthalten, in Abwesenheit wirksamer Mengen von Eisenkatalysatoren, wonach man nach Beendigung der Alkylierung das Phenolharz von verbliebenem 1.2.3.4-Tetrachlorbutan, überschüssigen Phenol und gegebenenfalls verwendetem Lösungsmittel auf an sich bekannte Weise trennt.

2. Verfahren nach Anspruch 1, wobei als Gemisch B) die bei der Chlorierung von Butadien anfallenden Nebenprodukte eingesetzt werden.

3. Verfahren nach Anspruch 1, wobei als Katalysator Zinksulfat eingesetzt wird.

4. Verfahren nach Anspruch 1, wonach die Komponenten A und B in einem Äquivalentverhältnis phenolisches OH zu funktionellen Gruppen des Gemischs B von 1:10 bis 10:1 eingesetzt werden.

5. Verfahren nach Anspruch 1, wonach die Reaktionstemperatur 40 bis 280° C beträgt.

6. Verfahren nach Anspruch 1, wonach man das Tetrachlorbutan aus dem Destillat enthaltend überschüssiges Phenol, Tetrachlorbutan und gegebenenfalls Lösungsmittel durch fraktionierte Destillation gewinnt.